# EUROPEAN PATENT SPECIFICATION

(11) **EP 3 522 815 B1**
(45) Date of publication and mention of the grant of the patent: **20.01.2021**
(21) Application number: 16816766.6
(22) Date of filing: 16.11.2016
(51) Int. Cl.: A61B 90/17, A61B 5/107, A61B 17/00, A61B 5/00

(54) **BREAST SIZE (VOLUME) MEASUREMENT APPARATUS**
GERÄT ZUM MESSEN VON BRUSTGRÖSSEN (-VOLUMINA)
APPAREIL DE MESURE LA TAILLE DU SEIN

(30) Priority: 05.10.2016 TR 201613969
(43) Date of publication of application: 14.08.2019
(73) Proprietor: Tezcan, Mustafa, Istanbul (TR)
(72) Inventor: Tezcan, Mustafa, Istanbul (TR)
(86) International application number: PCT/TR2016/050447
(87) International publication number: WO 2018/067081

(56) References cited:
- WO-A2-03/099101
- FR-A1- 2 520 999
- US-A- 4 219 029
- US-A- 5 772 654

## Description

### Specification

### Technical Field

This invention is a breast volume measurement apparatus which was developed in order to ensure symmetry between two breasts during therapeutic or cosmetic breast surgery and to guide the surgeon in terms of breast size reduction ratios.

### Background

Currently, women who wish to undergo breast surgery due to various reasons, therapeutic or cosmetic (breast reduction, augmentation or correction of deformation in the breast tissue etc.), obtain the desired appearance or medical outcome thanks to such surgical operations. However, surgeons currently conduct the necessary pre-operational measurements prior to breast surgery with the patient standing afoot, and there is lack of a proper apparatus for measurement of breast volume during the procedure itself, when the patient remains in recumbent position and measurements vary. Additionally, as tissue integrity is disturbed during an operation, pre-operational marking and measurement cannot provide the full effect intended. This leads to the surgeon essentially relying on one's own visual acuity as well as experience and skill in ensuring the desired symmetry between the two breasts. Considering specific issues such as carelessness, absent-mindedness and exhaustion, this situation where breast symmetry depends on the operating surgeon causes a certain risks, rendering some operations ineffective in terms of obtaining the desired symmetrical outcomes due to an auxiliary apparatus not being used during operation. Furthermore, due to the fact that there is currently no available apparatus to be used for the purpose grasping the entire breast and preventing tissues from slopping over, an additional supporting instrument may be needed. Prior art can be found in WO03/099101A2, or US5772654A.

### Summary

**The purpose of the invention is to enable measurement of breast sizes prior to and during cosmetic breast surgeries, allowing the surgeon to obtain the desired symmetrical balance between the breasts.**

**The purpose of this invention** is to allow the surgeon to grasp the entire breast, essentially preventing the breast from slopping over and eliminating the need for an additional supporting instrument during surgery.

**The purpose of this invention is to allow breast measurement during surgery while the patient is in recumbent position**, facilitating the task undertaken by the surgery and the surgical assistant.

**The purpose of this invention is to facilitate, in cases where a breast prosthesis is to be used, making a decision on prosthesis size prior to and during surgical *operation.***

### Brief Description of the Drawings

Figure 1 is a perspective view of the present invention; and
Figure 2 is a top view the Figure land;
Figure 3 is using of the device.

Numbers found on the figures describe the following:
1. volume measurement section
2. nipple spacing
3. opening for intervention
4. tissue control gaps
5. suture fixation channels

### Detailed Description

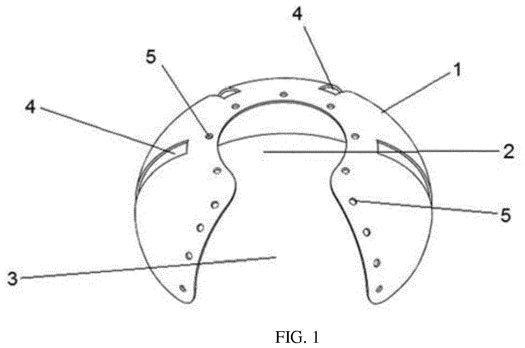

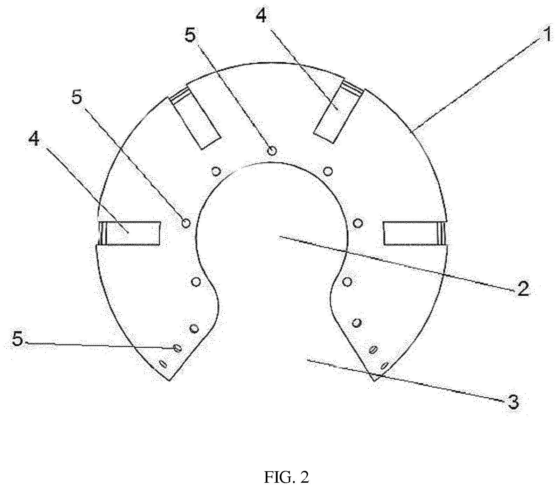

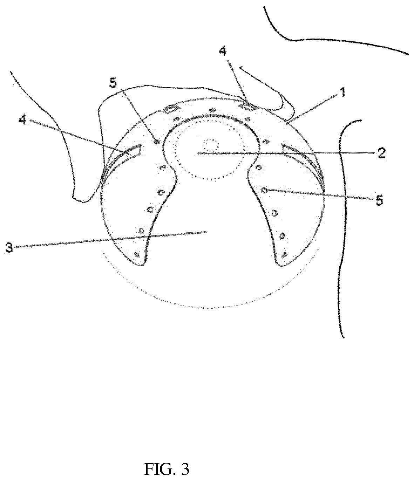

As shown in Figure 1 and Figure 2, a breast size (volume) measurement apparatus of the present invention includes a dome-like volume measurement section (1) made of semi-flexible metal or plastic derivatives. Using the nipple spacing (2), the surgeon may ensure that the patient's nipple is in this area, positioning the breast correctly. On the lateral surface of the volume measurement section (1), there is an opening for intervention (3) next to the nipple spacing (2). This opening for intervention (3) provides the volume measurement section (1) with a certain level of flexibility and allows necessary tissue adjustments etc. The volume measurement section (1) features control gaps (4), which enable easier handling of (Figure 3) the volume measurement section (1) as well as observing the breast tissue without it slopping over during surgery. The invention also features fixation channels (5) surrounding the nipple spacing (2) and the opening for intervention (3) above the volume measurement section (1). These fixation channels (5) allow fixation of the darker portion of the breast (nipple areola) using sutures, if deemed necessary.

The invention roughly plays the role of a mould during surgery, and thanks to its semi-flexible structure, it assumes the size/shape of the measured breast. This way, it can be placed on the other breast, which will be operated on, and both breasts can be symmetrically adjusted to each other after the surgeon performs the same operation as the first breast on the second one.

The lateral surface length of the invention, which extends from the dome-like volume measurement section (1) and the nipple spacing (2) to the base, gradually grows narrower to a certain extent as the opening for intervention is approached from the lateral side. This facilitates flexing of the dome-like volume measurement section (1). If desired, the lateral surface length, which extends from the dome-like volume measurement section (1) and the nipple spacing (2) to the base, may be redesigned so that it does not narrow down as the opening for intervention is approached from the rear side.

## Claims

1. A breast volume measurement apparatus, comprising:
- a dome-like volume measurement: section (1), wherein the volume measurement section is made of semi-flexible metal or plastic derivatives;
- a nipple spacing (2), wherein the nipple spacing is located in a middle of the volume measurement section, and configured to position a breast by placing a nipple in the nipple spacing;
- an opening for intervention (3), wherein the opening for intervention is on a lateral surface of the volume measurement section and next to the nipple spacing, and the opening for intervention provides the volume measurement section with a level of flexibility;
- a plurality of tissue control gaps (4), wherein the plurality of tissue control gaps are on the lateral surface of the volume measurement section, and the plurality of tissue control gaps facilitate a handling of the volume measurement section and prevent a breast tissue from slopping over during a surgery; and
- a plurality of fixation channels, (5), wherein the plurality of fixation channels surround the nipple spacing and the opening for intervention above the volume measurement section and allow a fixation of a darker portion of the breast (nipple areola) to the volume measurement section using sutures, **characterised in that** a lateral surface length, which extends from the dome-like volume measurement section (1) and the nipple spacing (2) to the base, gradually grows narrower as the opening for intervention (3) is approached from the rear side.

## Patentansprüche

1. A Brustvolumenmessgerät, bestehend aus
- einem kuppelartigen Volumenmessabschnitt (1),
wobei der Volumenmessabschnitt aus halbflexiblen Metali-oder Kunststoffderivaten besteht
- einem Nippelabstand (2)
wobei sich der Brustwarzenabstand in einer Mitte des Volumenmessabschnitts befindet und konfiguriert ist, um eine Brust zu positionieren, indem eine Brustwarze in den Brustwarzenabstand platziert wird;
- eine Interventionsöffnung (3)
wobei sich die Öffnung für den Eingriff auf einer Seitenfläche des Volumenmessabschnitts und neben dem Nippelabstand befindet und die Öffnung für den Eingriff dem Volumenmessabschnitt ein Maß an Flexibilität verleiht.
- eine Vielzahl von Gewebekontrolllücken (4),
wobei sich die Vielzahl von Gewebekontrolllücken auf der Seitenfläche des Volumenmessabschnitts befindet und die Vielzahl von Gewebekontrolllücken eine Handhabung des Volumenmessabschnitts erleichtern und verhindern, dass ein Brustgewebe während einer Operation umkippt; und
- Vielzahl von Fixierungskanälen (5),
wobei die Vielzahl von Fixierungskanälen den Brustwarzenabstand und die Öffnung zum Eingreifen Ober dem Volumenmessabschnitt umgeben und eine Fixierung eines dunkleren Teils der Brust (Brustwarzenhof) an dem Volumenmessabschnitt unter Verwendung von Nähten ermöglichen, die **dadurch gekennzeichnet sind, dass** eine laterale Oberflächenlänge, die sich vom kuppelartigen Volumenmessabschnitt (1) und dem Nippelabstand (2) bis zur Basis erstreckt, allmählich schmaler wird, wenn sich die Öffnung für den Eingriff (3) von der Rückseite nähert.

## Revendications

1. A Un appareil de mesure du volume du sein qui comprend:
- une section de mesure de volume ayant une forme de dôme (1),
dans lequel la partie de mesure de volume est en en plastiques dérivés ou en métal semi-flexible,
- un espacement des tétons (2)
dans lequel l'espacement des tétons est situé au milieu d'une section de mesure de volume et configuré pour positionner un sein en plaçant un téton dans l'espacement des tétons;
- une ouverture pour l'intervention (3)
dans lequel l'ouverture pour l'intervention se trouve à côté de l'espacement des tétons et sur une surface latérale de la section de mesure de volume et l'ouverture pour l'intervention permet à la partie de mesure de volume un niveau de flexibilité;
- une pluralité d'espaces de contrôle de tissu (4),
dans lequel la pluralité d'espaces de contrôle de tissu se trouvent sur la surface latérale de la section de mesure de volume et la pluralité d'espaces de contrôle de tissu facilitent une manipulation de la section de mesure de volume et empêchent un tissu mammaire de glisser pendant une intervention chirurgicale; et
- pluralité de canaux de fixation (5),
dans lequel la pluralité de canaux de fixation entourent l'espacement des mamelons et l'ouverture pour l'intervention au-dessus de la partie de mesure de volume et permettent à la section de mesure de volume une fixation d'une partie plus foncée du sein (aréole du mamelon) en utilisant: des sutures, **caractérisé en ce qu'**une surface latérale la longueur, qui s'étend de la section de mesure de volume en forme de dôme (1) et l'espacement des tétons (2) à la base, et à mesure que l'ouverture pour l'intervention (3) est approchée du côté arrière se rétrécit progressivement.
